# EUROPÄISCHE PATENTSCHRIFT

(11) **EP 3 773 264 B1**
(45) Veröffentlichungstag und Bekanntmachung des Hinweises auf die Patenterteilung: **11.12.2024**
(21) Anmeldenummer: 19714427.2
(22) Anmeldetag: 28.03.2019
(51) Int. Cl.: A61B 17/29

(54) **ELEKTRODEN-APPLIKATIONSINSTRUMENT**
ELECTRODE APPLICATION INSTRUMENT
INSTRUMENT D'APPLICATION D'ÉLECTRODES

(30) Priorität: 28.03.2018 DE 202018101753 U
(43) Veröffentlichungstag der Anmeldung: 17.02.2021
(73) Patentinhaber: Tuebingen Scientific Medical GmbH, 72076 Tuebingen (DE)
(72) Erfinder: BRAUN, Marcus, 71093 Weil im Schönbuch (DE)
(74) Vertreter: Winter, Brandl - Partnerschaft mbB
(86) Internationale Anmeldenummer: PCT/EP2019/057946
(87) Internationale Veröffentlichungsnummer: WO 2019/185845

(56) Entgegenhaltungen:
- EP-A2- 1 886 630
- WO-A1-2004/098701
- US-A- 5 752 973

## Beschreibung

Die vorliegende Erfindung betrifft ein Elektroden-Applikationsinstrument der minimalinvasiven Bauart gemäß dem Oberbegriff des Schutzanspruchs 1.

### Hintergrund der Erfindung

Beispielsweise im Falle von Herzrhythmusstörungen werden häufig dem Patienten Herzschrittmacher implantiert, welche über elektrische Leitungen sowie daran endseitig montierte Elektroden mit bestimmten Bereichen des Herzens elektrisch verbunden sind. Entscheidend ist es hierbei, dass die Elektroden korrekt und sicher am Herzen installiert/gesetzt/verankert werden. Zu diesem Zweck weisen die Elektroden schrauben- oder korkenzieherartige Fortsätze/Anker auf, die in das Muskelgewebe des Herzens eingedreht werden.

Für die Implantation derartiger Elektroden haben sich in der Vergangenheit zwei Vorgehensweisen etabliert. Zum einen können Elektroden dieser Gattung per Katheter über die Halsschlagader in das Innere des Herzens eingeführt und dort fixiert werden. Dies hat den Vorteil, dass der Patient nur geringfügig operativ verletzt wird (geringes Operationstrauma) und daher das Infektionsrisiko weitestgehend minimiert werden kann. Jedoch hat diese Vorgehensweise den Nachteil, dass die Positionierung der Elektroden schwierig und teilweise auch ungenau ist. Zum anderen können Elektroden dieser Gattung minimalinvasiv mittels eines chirurgischen Instruments der Schaftbauart an die Außenseite des Herzens gesetzt werden. Zu diesem Zweck wird beispielsweise ein minimalinvasiver Patientenzugang geschaffen, durch welchen der Instrumentenschaft des chirurgischen Instruments eingeführt werden kann.

### Stand der Technik

Aus dem Stand der Technik beispielsweise gemäß der US 7,544, 197B2 ist ein Elektroden-Applikationsinstrument der Schaftbauart bekannt. Dieses Instrument hat einen Handgriff, der an einem proximalen Ende eines Instrumentenschafts angeschlossen ist, an dessen distalem Ende ein Effektor schwenkbar sowie drehbar gelagert ist. Der Effektor bildet ein Maulteil/Instrumentenkopf zur Aufnahme einer Elektrode für deren Applikation an der Außenwand eines Herzens. Das Maulteil ist in Form einer Tulpe aufgebaut mit drei oder vier in Umfangsrichtung beabstandeten federelastischen Laschen, die eine Art Aufnahmering bilden, in welchen eine Elektrode eingeklemmt werden kann. Darüber hinaus bilden sich zwischen den Laschen stirnseitige halbrunde Ausnehmungen oder Einkerbungen, durch welche die Elektrodenlitzen geführt werden können, wenn die Elektrode in dem Aufnahmering eingeklemmt ist.

Es hat sich gezeigt, dass ein derart gestaltetes Maulteil zwar die Elektrode sicher halten und Einschrauben kann, jedoch die Elektrode nach deren Befestigung am Herzmuskel nicht einfach freigeben kann.

### Kurzbeschreibung der Erfindung

Angesichts dieser Problematik ist es eine Aufgabe der vorliegenden Erfindung, ein Elektroden-Applikationsinstrument bereitzustellen, mit welchem das Applizieren von Elektroden insbesondere am Herzmuskel in einfacher und sicherer Weise durchgeführt werden kann.

Diese Aufgabe wird durch ein chirurgisches Instrument, insbesondere Elektroden-Applikationsinstrument der minimalinvasiven Bauart mit den Merkmalen des Schutzanspruchs 1 gelöst. Vorteilhafte Weiterbildungen der Erfindung sind Gegenstand der abhängigen Ansprüche.

Genauer betrifft die vorliegende Offenbarung ein chirurgisches Elektroden-Applikationsinstrument der minimalinvasiven Bauart mit einem Effektor, der einen am distalen Ende eines Instrumentenschafts um dessen Längsachse drehbar sowie schwenkbar gelagerten Instrumentenkopf bildet, an welchem zwei sich gegenüberliegende sowie scheren- oder zangenartig aufeinander zu bewegbare Branchen gelagert sind, von denen zumindest eine Branche mit einer stirnseitigen teilkreisförmigen Einkerbung für die Aufnahme einer Elektrodenlitze ausgebildet ist und die zumindest abschnittsweise jeweils im Querschnitt sichelförmig geformt sind, wobei die einander zugewandten Klemmseiten im sichelförmig geformten Abschnitt jeder Branche jeweils eine Anzahl, vorzugsweise Mehrzahl, von in Branchenlängsrichtung beabstandeten sowie in Branchenquerrichtung vorzugsweise parallel verlaufender Rillen oder Hinterschneidungen aufweisen, wobei zumindest eine oder beide Branchen für deren aktive Betätigung mit Anlenkstellen für einen Betätigungsmechanismus ausgebildet sind, der eine Anzahl von Betätigungselementen aufweist, von welchen eines über einen Getriebezug mit den Branchen an der entsprechenden Anlenkstelle gekoppelt ist, derart, dass über das eine Betätigungselement eine Betätigungskraft sowohl in Öffnungs- als auch in Schließrichtung aufbringbar ist.

Der Kern der vorliegenden Erfindung besteht demzufolge in der besonderen Ausbildung des Effektors bzw. Maulteils mit zwei sich gegenüberliegenden sowie zangenartig aufeinander zu bewegbaren Branchen, von denen zumindest eine Branche (vorzugsweise beide) mit einer stirnseitigen teilkreisförmigen Einkerbung (für die Aufnahme einer Elektrodenlitze) ausgebildet ist und die jeweils im Querschnitt (wenn in Richtung proximal betrachtet) sichelförmig gebogen bzw. geformt sind, wodurch sich an den einander zugewandten Innenseiten der Branchen (zumindest im Bereich eines Eingriffsabschnitts der Branche) eine in Längsrichtung (Richtung proximal) sich erstreckende Rinnenform ergibt. Die einander zugewandten Innen-/Klemmseiten jeder Branche weisen zudem jeweils eine Anzahl von in axialer Richtung beabstandeten (Innenumfangs-)Rillen oder Hinterschneidungen auf, die bevorzugt gleiche Radien bzw. Rillentiefen haben und zur (axialen) Aufnahme einer Elektrode dienen (d.h. zur Aufnahme der in der Regel knopf- oder tellerförmigen Elektrode nicht von der Seite her sondern von oben).

An dieser Stelle sei darauf hingewiesen, dass bereits eine einzige Rille für das korrekte Halten der Elektrode ausreicht, nämlich derart, dass der Korkenzieher-artige Fortsatz einer allgemein bekannten Elektrode axial zum Instrumentenkopf/Maulteil ausgerichtet ist, wobei eine Mehrzahl von Rillen vorteilhaft dahingehend ist, dass hierdurch ein höheres Drehmoment auf die Elektrode übertragbar ist.

Die Branchen des Maulteils sind gelenkig/schwenkbar an einem Instrumentenkopf gelagert und über eine Betätigungseinrichtung/Getriebezug mit einer Handhabe gekoppelt. Der Instrumentenkopf ist dabei schwenkbar sowie (um dessen Längsachse) drehbar an einem distalen Ende eines Instrumentenschafts gehalten. Die Handhabe ist an einem proximalen Ende des Instrumentenschafts angeordnet, wobei die Betätigungseinrichtung/Getriebezug (zumindest teilweise) innerhalb des Instrumentenschafts vorgesehen ist. Die Handhabe hat eine Anzahl von Betätigungselementen, die in einem Handgriff integriert sind, um in jeweils separater Weise den Instrumentenkopf um dessen Längsachse zu drehen, diesen bezüglich des Instrumentenschafts abzuwinkeln/zu verschwenken und die Branchen zu öffnen/ zu schließen. Ein derartiges Grundkonzept eines chirurgischen Instruments der minimalinvasiven Bauart insbesondere die Konstruktion der Handhabe sowie der Aufbau des Instrumentenkopfs/Maulteils sind beispielsweise aus den Druckschriften DE 103 24 844 A1, DE 103 30 604 A1, DE 10 2009 001 278 A1 und WO 2004/098 701 A bekannt.

Die beiden Branchen des Maulteils sind vorzugsweise identisch zueinander ausgebildet und weisen weiter bevorzugt einen in Draufsicht oder Unteransicht (Blickrichtung auf die Klemmseite des Eingreifabschnitts) achsversetzten Branchenhals auf, der distal in einen achszentrisch angeordneten Eingreifabschnitt/Greifabschnitt einstückig übergeht. Der Eingreifabschnitt weist hierbei die im Querschnitt sichelförmig gebogene Kontur gemäß vorstehender Beschreibung auf, ist an seiner radialen Außenseite vorzugsweise glattflächig ausgebildet und an seiner radialen Innenseite mit den vorstehend beschriebenen axialbeabstandeten sowie in Umfangsrichtung sich erstreckenden Rillen versehen.

Der Branchenhals ist vorzugsweise plättchenartig geformt mit einer Durchgangsbohrung am distalen Übergangsbereich zwischen Branchenhals und ein Greifabschnitt sowie weiter bevorzugt einem axial sich erstreckenden, jedoch schräg zur Längsrichtung angestellten Langloch im proximalen Endabschnitt des Branchenhalses. Der plättchenartige Branchenhals ist dabei bevorzugt um ca. 90° zum Greifabschnitt verdreht, derart, dass die Durchgangsbohrung und das Langloch in Querrichtung der Branche (längs der Innenumfangsrillen) ausgerichtet sind. Auf diese Weise lassen sich die Branchen in einem Instrumentenkopf scherenartig schwenkbar am jeweiligen Durchgangsloch montieren, wobei das jeweilige Langloch eine Art Schiebekulisse bildet, derart, dass beispielsweise ein im Instrumentenkopf in Längsrichtung gleitgeführter Querstift das Langloch durchgreift und bei dessen Längsbewegung die Branche infolge der Schrägstellung des Langlochs um das Scharnier dreht. Dabei sei darauf hingewiesen, dass die Translation der Schiebebewegung des Branchen-Getriebezugs auch durch einen anderen Mechanismus (z.B. über Pleuel) in eine Schwenkbewegung der Branchen transformierbar ist.

### Kurzbeschreibung der Figuren

Die Erfindung wird nachstehend anhand eines bevorzugten Ausführungsbeispiels unter Bezugnahme auf die begleitenden Figuren näher erläutert.
Figur 1 zeigt ein chirurgisches Elektroden-Applikationsinstrument gemäß einem bevorzugten Ausführungsbeispiel der vorliegenden Erfindung,
Figur 2 zeigt den Instrumentenkopf des chirurgischen Instruments gemäß Figur 1,
Figur 3 zeigt einen Längsschnitt des Instrumentenkopfs gemäß Figur 2,
Figur 4 zeigt die Seitenansicht einer Instrumentenbranche gemäß einem bevorzugten Ausführungsbeispiel der Erfindung, wie sie am Instrumentenkopf gemäß der Figur 3 Verwendung findet,
Figur 5a bis 5d zeigen den Eingriffsabschnitt einer Instrumentenbranche gemäß der vorliegenden Erfindung im Querschnitt, in der unter Ansicht sowie im Längsschnitt.

### Figurenbeschreibung

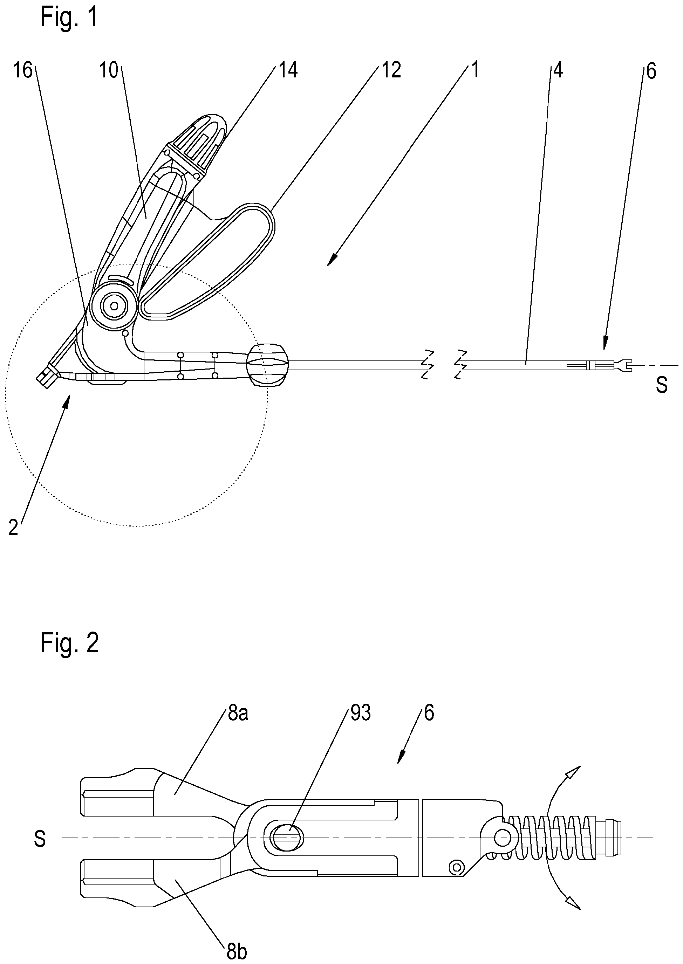

Das in Figur 1 gezeigte chirurgische Instrument 1 zur Applikation von Elektroden (nicht weiter dargestellt) beispielsweise an einem Herzen eines Patienten hat eine Handhabe 2 in Form eines Griffstücks, das an dem proximalen Ende eines Instrumentenschafts 4 montiert ist. Am distalen Ende des Instrumentenschafts 4 ist ein Effektor in Form eines Instrumentenkopfes 6 montiert, der drehbar um dessen Längsachse S sowie schwenkbar bezüglich der Längsachse des Instrumentenschafts an diesem gelagert ist. Im/am Instrumentenkopf 6 sind zwei Branchen 8a, 8b scheren- bzw. zangenartig gelagert (siehe insbesondere Fig. 2), derart, dass diese zusammen mit dem Instrumentenkopf 6 gedreht sowie geschwenkt werden können und unabhängig hiervon geöffnet und geschlossen werden können.

Am Griffstück/Instrumentengriff 2 sind eine Anzahl von Betätigungselementen (vorzugsweise drei Betätigungselemente) 10, 12, 14 angeordnet. Zunächst hat der Instrumentengriff 2 eine Art Steuerknüppel 10, der schwenk- bzw. abklappbar an einer Griffbasis 16 gelagert ist und über einen im Instrumentenschaft 4 geführten Getriebebezug mit dem Instrumentenkopf 6 derart gekoppelt ist, dass eine Schwenkbewegung des Steuerknüppels 10 bezüglich der Griffbasis 16 in eine Schwenkbewegung des Instrumentenkopfs 6 bezüglich des Instrumentenschafts 4 transformiert wird. Des Weiteren ist an dem Steuerknüppel 10 ein Schwenkhebel 12 angelenkt, dessen Schwenkbewegung bezüglich des Steuerknüppels 10 über einen ebenfalls im Instrumentenschaft 4 geführten separaten Getriebezug auf die Branchen 8a, 8b übertragen wird, um diese scheren-bzw. zangenartig zu öffnen und zu schließen. Schließlich ist an dem freien Ende des Steuerknüppels 10 ein Drehknopf 14 gelagert, dessen Rotation über einen ebenfalls separat im Instrumentenschaft 4 angeordneten Getriebezug auf den Instrumentenkopf 6 einwirkt, um diesen um seine Längsachse zu drehen.

Die Konstruktion der Handhabe 2, der jeweiligen separaten Getriebezüge sowie des Instrumentenkopfs 6 sind aus dem Stand der Technik gemäß der eingangs genannten Druckschriften der vorliegenden Anmelderin bekannt, sodass an dieser Stelle auf diese Druckschriften verwiesen werden kann.

Gemäß dem vorliegenden Ausführungsbeispiel der Erfindung unterscheidet sich das chirurgische Instrument zur Applikation von Elektroden gegenüber den bisher bekannten chirurgischen Instrumenten der minimalinvasiven Bauart jedoch im Wesentlichen durch die Konstruktion der erfindungsgemäß verwendeten Branchen 8a, 8b.

Elektroden bekannter Bauart zur Montage beispielsweise am Herzen eines Patienten weisen in der Regel einen münzen- oder knopfartig geformten (flachen und runden) Elektrodenkopf auf, an dessen flacher Unterseite eine Korkenzieher-artige Schraubspirale/Anker angeordnet ist, die über eine elektrische Litze (Kabel) beispielsweise mit einem Herzschrittmacher verbunden werden kann. Eine solche Elektrode muss zu deren Befestigung in den Herzmuskel eingedreht werden, wobei es für deren sicheren Halt im vergleichsweise schwammigen Herzmuskelgewebe entscheidend ist, dass der Einschraubvorgang möglichst axial längs der Schraubspirale erfolgt, ohne dabei eine Schaukel- oder Taumelbewegung auszuführen. D.h., dass die Elektrode so ergriffen und gehalten werden muss, dass sich die Schraubspirale im Wesentlichen axial zur Längsachse des Instrumentenkopfs 6 erstreckt.

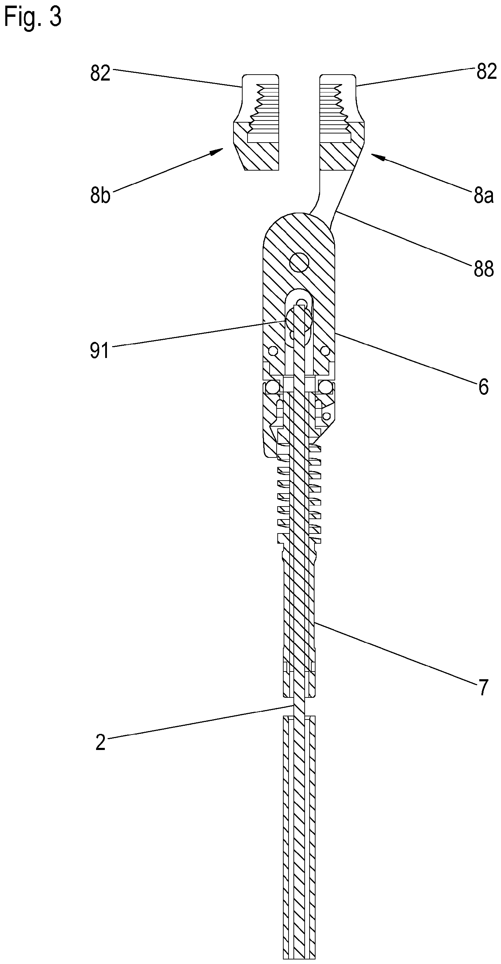

Zur Erreichung dieses Effekts weisen die im erfindungsgemäßen Instrument 1 vorgesehenen Branchen 8a, 8b eine Konstruktion auf, wie sie insbesondere in den Figuren 2 bis 5 dargestellt ist.

Zunächst ist darauf hinzuweise, dass die scheren- bzw. zangenartig am Instrumentenkopf 6 gelagerten Branchen 8a, 8b vorzugsweise baugleich sind, sodass im Nachfolgenden nur eine der beiden Branchen beschrieben werden muss.

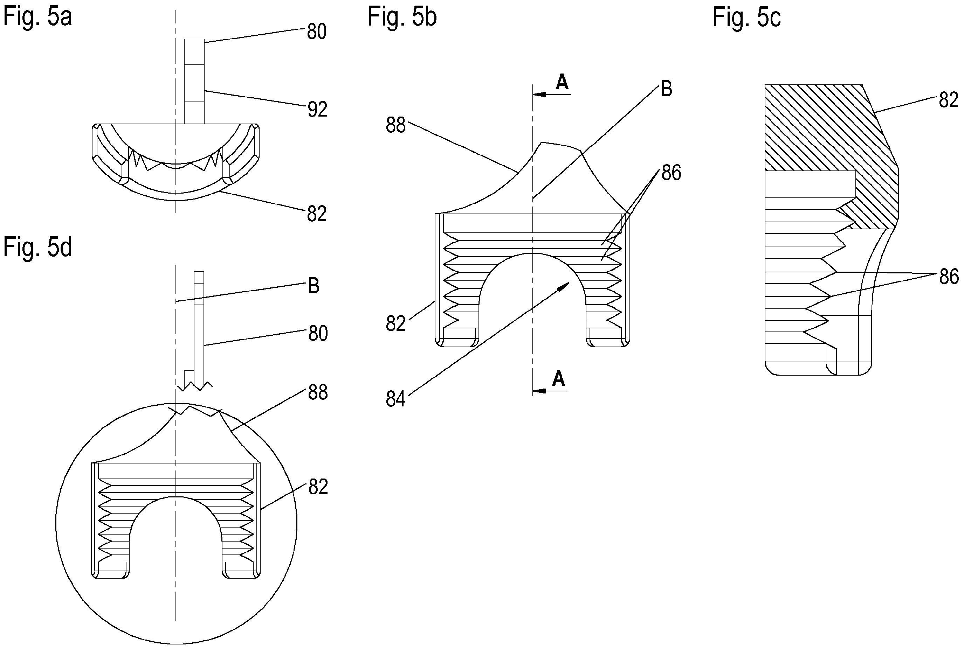

Jede Branche 8a, 8b hat demzufolge einen proximalen Branchenhals 80 und einen distal sich stoffeinstückig (über einen zwischenliegenden Übergangsbereich) daran anschließenden Greifabschnitt 82. Der Greifabschnitt 82 wird durch eine im Querschnitt (in Branchenlängsrichtung gesehen) sichel- oder rinnenförmige Platte gebildet (Siehe Fig:5a), die an ihrer distalen Stirnseite eine teilkreis-/halbkreisförmige Einkerbung 84 (siehe Fig. 5b) aufweist, die im Wesentlichen mittig bezüglich der Branchenlängsachse B angeordnet ist. Eine (radiale) Außenseite des Greifabschnitts 82 ist vorzugsweise glattflächig gestaltet, wohingegen eine (radiale) Innenseite des Greifabschnitts (nachfolgend als Klemmseite bezeichnet) ein Rillenprofil aufweist.

Im bevorzugten Ausführungsbeispiel besteht das Rillenprofil aus einer Anzahl von in Branchenachsrichtung beabstandeten Rillen 86, die sich In Querrichtung bzw. in Umfangsrichtung des Greifabschnitts 82 und damit im Wesentlichen senkrecht zur Branchenlängsachse B über den gesamten Greifabschnitt 82 hinweg erstrecken. Des Weiteren weisen alle Rillen 86 bevorzugt den gleichen Radius bzw. die gleiche Rillentiefe auf. Auch die Rillenbreite bleibt vorzugsweise gleich, kann sich aber ausgehend von distal in Richtung proximal verändern.

Der Branchenhals 80 weist eine platten- oder plättchenartige Form auf und ist gegenüber dem Greifabschnitt 82 um im Wesentlichen 90° um die Branchenlängsachse gedreht. D.h. während der Greifabschnitt 82 beispielsweise quasi horizontal ausgerichtet ist, erstreckt sich der plättenförmige Branchenhals 80 im Wesentlichen vertikal. An einem distalen Übergangsbereich 88 zwischen Branchenhals 80 und Greifabschnitt 82 ist eine Durchgangsbohrung 90 ausgebildet, die sich infolge der 90° Drehung im Wesentlich längs (tangential) der Rillen 86 erstreckt. An einem proximalen Endabschnitt des Branchenhalses 82 ist ferner ein Langloch 92 ausgeformt, das sich längs der Branchenlängsachse erstreckt, jedoch zusätzlich in einem (spitzen) Winkel (beispielsweise 15°-20°) zur Branchenlängsachse angestellt ist.

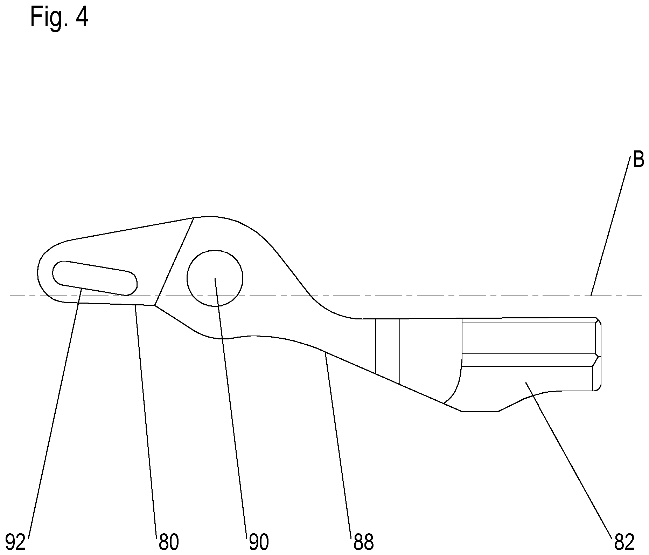

Wie insbesondere in der Fig. 4 dargestellt ist, verlaufen die Branchenlängsachse im Bereich des Branchenhalses 80 sowie die Branchenlängsachse im Bereich des Greifabschnitts 82 im Wesentlichen parallel zueinander, wohingegen die Branchenlängsachse B im Übergangsbereich 88 zwischen Branchenhals 80 und Greifabschnitt 82 in der Seitenansicht schräg hierzu angestellt ist, wodurch sich in der Seitenansicht ein S-förmiger Branchenverlauf ergibt. Demzufolge bilden die beiden scheren- bzw. zangenartig am Instrumentenkopf 6 montierten Branchen 8a, 8b zwischen den sich gegenüberliegenden Klemmseiten einen Klemmspalt, wenn sich die beiden Branchen 8a, 8b in einer Stellung befinden, in welcher die Klemmseiten parallel zueinander ausgerichtet sind. Diese Stellung ist beispielsweise in der Fig. 2 dargestellt.

Wie schließlich aus den Fig. 5b und 5d besonders gut zu entnehmen ist, sind der Branchenhals 80 und der Greifabschnitt 82 in Unteransicht (d.h. mit Blick auf die Klemmseite des Greifabschnitts 82) asymmetrisch angeordnet. In anderen Worten ausgedrückt ist der Branchenhals 80 bezüglich der Mittel- bzw. Längsachse (Branchenlängsachse B) des Greifabschnitts 82 parallelversetzt, derart, dass bei einem aneinander Liegen zweier Branchen 8a, 8b im Bereich der Branchenhälse 82 die beiden sich gegenüberliegenden Klemmseiten im Wesentlichen fluchten bzw. überlappen.

### Funktionsweise

Für ein Applizieren einer Elektrode beispielsweise an der Außenseite eines Patientenherzens wird die Elektrode randseitig mit den scherenartig am Instrumentenkopf montierten Branchen 8a, 8b ergriffen, derart, dass der Elektrodenrand in den selben Rillen an den sich gegenüberliegenden Branchen eingreift. Auf diese Weise ist gewährleitet, dass sich die Elektrode im Wesentlichen senkrecht zur Instrumentenkopfachse ausrichtet. Dabei ist darauf zu achten, dass der Elektrodenrand in jener Rille mit bestimmten Radius aufgenommen ist, dass sich die beiden Branchen beim Festhalten der Elektrode im Wesentlichen parallel zueinander ausrichten.

Der Greifvorgang erfolgt im Wesentlichen durch Betätigen des Hebels 12, wodurch über den zugehörigen Getriebezug im Instrumentenschaft 4 ein im Instrumentenkopf 6 axial verschiebbar gelagerter Zapfen oder Stift 91 längsverschoben wird, der wiederum in die Langlöcher 92 eingreift. Aufgrund der Schrägstellung der Langlöcher 92 bezüglich der Branchenlängsachse B bzw. der Instrumentenkopf-Längsachse S, wirken die Langlöcher 92 als Führungskulissen, derart, dass bei dem Axialverschieben des Zapfens/Stifts 91 längs der Langlöcher 92 die Branchen 8a, 8b gleichzeitig um einen in die Durchgangsbohrungen 90 eingesteckten Schwenkzapfen 93 gedreht werden.

Nunmehr kann die Elektrode in den Herzmuskel eingedreht werden, indem der Instrumentenkopf 6 so geschwenkt wird, dass sich dieser im Wesentlichen senkrecht zur Herzaußenseite ausrichtet und anschließend der Instrumentenkopf sowie die das Maulteil bildenden Branchen 8a, 8b gedreht werden. Sobald die Elektrode fest in den Herzmuskel eingedreht ist, können die Branchen 8a, 8b geöffnet und damit die Elektrode freigegeben werden.

## Patentansprüche

1. Chirurgisches Elektroden-Applikationsinstrument der minimalinvasiven Bauart mit einem Effektor, der einen am distalen Ende eines Instrumentenschafts (4) um dessen Längsachse drehbar sowie schwenkbar gelagerten Instrumentenkopf (6) bildet, an welchem zwei sich gegenüberliegende sowie scheren- oder zangenartig aufeinander zu bewegbare Branchen (8a, 8b) gelagert sind, von denen zumindest eine Branche (8a) mit einer stirnseitigen teilkreisförmigen Einkerbung (84) für die Aufnahme einer Elektrodenlitze ausgebildet ist und die zumindest abschnittsweise jeweils im Querschnitt sichelförmig geformt sind, wobei die einander zugewandten Klemmseiten im sichelförmig geformten Abschnitt jeder Branche (8a, 8b) jeweils eine Anzahl, vorzugsweise Mehrzahl, von in Branchenlängsrichtung beabstandeten sowie in Branchenquerrichtung vorzugsweise parallel verlaufender Rillen oder Hinterschneidungen (86) aufweisen, wobei zumindest eine oder beide Branchen (8a, 8b) für deren aktive Betätigung mit Anlenkstellen für einen Betätigungsmechanismus ausgebildet sind, der eine Anzahl von Betätigungselementen (12) aufweist, von welchen eines über einen Getriebezug mit den Branchen (8a, 8b) an der entsprechenden Anlenkstelle gekoppelt ist, derart, dass über das eine Betätigungselement (12) eine Betätigungskraft sowohl in Öffnungs- als auch in Schließrichtung aufbringbar ist, wobei die Anzahl von Betätigungselementen (12) am proximalen Ende des Instrumentenschafts (4) angebracht ist und über den Getriebezug, welcher zumindest teilweise innerhalb des Instrumentenschafts (12) angeordnet ist, mit dem Instrumentenkopf (6) gekoppelt ist.

2. Chirurgisches Elektroden-Applikationsinstrument nach Anspruch 1, **dadurch gekennzeichnet, dass** das mit den Branchen (8a, 8b) gekoppelte Betätigungselement (12) ein manuell betätigbarer Schwenkhebel (12) ist, der an einem Instrumentengriff (2) angelenkt ist und der dafür vorgesehen und ausgebildet ist, die Betätigungskraft sowohl in Öffnungs- als auch in Schließrichtung manuell aufzubringen.

3. Chirurgisches Elektroden-Applikationsinstrument nach Anspruch 1 oder 2, **dadurch gekennzeichnet, dass** die beiden Branchen (8a, 8b) baugleich zueinander ausgebildet sind und jeweils einen in Draufsicht achsversetzten Branchenhals (80) aufweisen, der distal in einen achszentrisch angeordneten Eingreifabschnitt (82) einstückig übergeht.

4. Chirurgisches Elektroden-Applikationsinstrument nach Anspruch 3, **dadurch gekennzeichnet, dass** der Eingreifabschnitt (82) die im Querschnitt sichelförmig gebogene Kontur aufweist, an seiner radialen Außenseite glattflächig ausgebildet ist und an seiner radialen Innenseite mit den axialbeabstandeten sowie quer in Umfangsrichtung sich erstreckenden Rillen (86) versehen ist.

5. Chirurgisches Elektroden-Applikationsinstrument nach Anspruch 3 oder 4, **dadurch gekennzeichnet, dass** der Branchenhals (80) plättchenartig geformt ist mit einer Durchgangsbohrung (90) an einem distalen Übergangsbereich (88) zwischen Branchenhals (80) und Greifabschnitt (82) sowie einem axial sich erstreckenden Langloch (92) im proximalen Endabschnitt des Branchenhalses (80), wobei das Langloch (92) schräg zur Längsachse (B) der jeweiligen Branche (8a, 8b) angestellt ist.

6. Chirurgisches Elektroden-Applikationsinstrument nach Anspruch 5, **dadurch gekennzeichnet, dass** der plättchenartige Branchenhals (80) bezüglich des Greifabschnitts (82) ca. 90° um die Branchenlängsachse (B) gedreht ist, derart, dass die Durchgangsbohrung (90) und das Langloch (92) im Wesentlichen längs der Rillen (86) an der Klemmseite des Greifabschnitts (82) ausgerichtet sind.

## Claims

1. A surgical electrode application instrument of the minimally invasive type, the instrument comprising an effector forming an instrument head (6) arranged at the distal end of an instrument shaft (4) and mounted so as to be rotatable about the longitudinal axis thereof and in a pivotable manner, on which effector two opposite branches (8a, 8b) are mounted, which can be moved towards each other in a scissors-like or forceps-like manner, of which at least one branch (8a) is formed with a part-circular notch (84) at an end face for receiving an electrode litz wire, and the cross-section of which is respectively sickle-shaped at least in portions, wherein the mutually facing clamping sides in the sickle-shaped portion of each branch (8a, 8b) each have a number, preferably a plurality, of grooves or undercuts (86) which are spaced apart in a longitudinal direction of the branch and are preferably parallel in a transverse direction of the branch, wherein at least one or both branches (8a, 8b), for active actuation thereof, are formed with articulation points for an actuating mechanism that has a number of actuating elements (12), one of which is coupled via a gear train to the branches (8a, 8b) at the respective articulation point, such that, via an actuating element (12), an actuating force can be applied both in an opening and in a closing direction, wherein the number of actuating elements (12) is mounted on the proximal end of the instrument shaft (4) and is coupled to the instrument head (6) via the gear train, which is arranged, at least partially, inside the instrument shaft (12).

2. The surgical electrode application instrument according to claim 1, **characterized in that** the actuating element (12) coupled to the branches (8a, 8b) is a manually operable pivot lever (12) which is hinged to an instrument handle (2) and which is provided and configured to manually apply the actuating force both in an opening as well as in an closing direction.

3. The surgical electrode application instrument according to claim 1 or 2, **characterized in that** the two branches (8a, 8b) are identical in construction to each other and each have a branch neck (80) which is axially offset in a plan view and merges distally into an engagement portion (82) arranged axially centrally in one piece.

4. The surgical electrode application instrument according to claim 3, **characterized in that** the engagement portion (82) has a sickle-shaped contour in cross-section, is formed with a smooth surface on its radial outside, and is provided on its radial inside with grooves (86) axially spaced apart and extending transversely in a circumferential direction.

5. The surgical electrode application instrument according to claim 3 or 4, **characterized in that** the branch neck (80) is shaped like a platelet with a through bore (90) on a distal transition portion (88) between the branch neck (80) and the engagement portion (82) and an axially extending elongated hole (92) in the proximal end portion of the branch neck (80), wherein the elongated hole (92) is set at an angle to a longitudinal axis (B) of a respective branch of the two branches (8a, 8b).

6. The surgical electrode application instrument according to claim 5, **characterized in that** the platelet-shaped branch neck (80) is rotated by approximately 90° about the longitudinal branch axis (B) with respect to the engagement portion (82) such that the through bore (90) and the elongated hole (92) are oriented substantially along the grooves (86) on the clamping side of the engagement portion (82).

## Revendications

1. Instrument chirurgical d'application d'électrodes de type à invasion minimale avec un effecteur qui forme une tête d'instrument (6) montée à l'extrémité distale d'une tige d'instrument (4) de manière rotative et pivotante autour de son axe longitudinal, tête d'instrument sur laquelle deux branches (8a, 8b) sont montées opposées l'une à l'autre et pouvant se déplacer l'une vers l'autre à la manière de ciseaux ou de pinces, dont au moins une branche (8a) est conçue avec une encoche (84) frontale en forme de cercle partiel pour la réception d'un toron d'électrode et qui sont formées au moins par sections respectivement en forme de croissant dans la section transversale, dans lequel les côtés de serrage tournés l'un vers l'autre dans la section formée en forme de croissant de chaque branche (8a, 8b) présentent respectivement un nombre, de préférence une pluralité, de côtés de serrage, de rainures ou de contre-dépouilles (86) espacés dans le sens longitudinal de la branche et s'étendant de préférence parallèlement dans le sens transversal de la branche, dans lequel au moins une ou les deux branches (8a, 8b) sont conçues pour leur actionnement actif avec des points d'articulation pour un mécanisme d'actionnement qui présente un nombre d'éléments d'actionnement (12) dont un est couplé par un train d'engrenages aux branches (8a, 8b) au niveau du point d'articulation correspondant, de sorte qu'une force d'actionnement puisse être appliquée par l'intermédiaire de l'un des éléments d'actionnement (12) à la fois dans le sens d'ouverture et dans le sens de fermeture, dans lequel le nombre d'éléments d'actionnement (12) est appliqué à l'extrémité proximale de la tige d'instrument (4) et est couplé à la tête d'instrument (6) par l'intermédiaire du train d'engrenages qui est disposé au moins partiellement à l'intérieur de la tige d'instrument (12).

2. Instrument chirurgical d'application d'électrodes selon la revendication 1, **caractérisé en ce que** l'élément d'actionnement (12) couplé aux branches (8a, 8b) est un levier pivotant (12) pouvant être actionné manuellement, qui est articulé sur un manche d'instrument (2) et qui est prévu et conçu pour appliquer manuellement la force d'actionnement à la fois dans la direction d'ouverture et dans la direction de fermeture.

3. Instrument chirurgical d'application d'électrodes selon la revendication 1 ou 2, **caractérisé en ce que** les deux branches (8a, 8b) sont de construction identique l'une à l'autre et présentent respectivement un col de branche (80) à axe décalé en vue de dessus et qui devient distalement d'un seul tenant une section d'engagement (82) disposée de manière centrée sur l'axe.

4. Instrument chirurgical d'application d'électrodes selon la revendication 3, **caractérisé en ce que** la section d'engagement (82) qui présente le contour courbé en forme de croissant dans la section transversale est conçue avec une surface lisse sur son côté extérieur radial et est dotée sur son côté intérieur radial des rainures (86) espacées axialement et s'étendant transversalement dans la direction circonférentielle.

5. Instrument chirurgical d'application d'électrodes selon la revendication 3 ou 4, **caractérisé en ce que** le col de branche (80) a une forme de plaquette avec un alésage traversant (90) sur une zone de transition distale (88) entre le col de branche (80) et la section de préhension (82), ainsi qu'un trou oblong (92) s'étendant axialement dans la section d'extrémité proximale du col de branche (80), dans lequel le trou oblong (92) est incliné par rapport à l'axe longitudinal (B) de la branche respective (8a, 8b).

6. Instrument chirurgical d'application d'électrodes selon la revendication 5, **caractérisé en ce que** le col de branche (80) en forme de plaquette est tourné d'env. 90° autour de l'axe longitudinal de branche (B) par rapport à la section de préhension (82), de sorte que l'alésage traversant (90) et le trou oblong (92) soient orientés sensiblement le long des rainures (86) sur le côté de serrage de la section de préhension (82).
